# EUROPEAN PATENT APPLICATION

(11) **EP 1 550 442 A1**
(43) Date of publication of application: **06.07.2005**
(21) Application number: 03754031.7
(22) Date of filing: 08.10.2003
(51) Int. Cl.: A61K 31/198, A61P 9/00, A61P 25/28, A61P 43/00

(54) **COMPOSITION FOR PREVENTING/TREATING THE EXPRESSION OF CLINICAL SYMPTOM IN DISEASE CAUSED BY MITOCHONDRIAL DYSFUNCTION**

(30) Priority: 11.10.2002 JP 2002299575; 26.12.2002 JP 2002378176
(71) Applicant: Koga, Yasutoshi, Kurume-shi, Fukuoka 830-0003 (JP); Ajinomoto Pharma Co., Ltd., Chuo-ku, Tokyo 104-0028 (JP)
(72) Inventor: KOGA, Yasutoshi, Kurume-shi, Fukuoka 830-0003 (JP); NAKANISHI, Masato, Ajinomoto Pharma Co., Ltd., Chuo-ku, Tokyo 104-0028 (JP)
(74) Representative: Nicholls, Kathryn Margaret
(86) International application number: PCT/JP2003/012891
(87) International publication number: WO 2004/032917

(57) **Abstract**

In this application is disclosed an excellent composition for preventing and/or treating the expression of clinical symptoms in a disease caused by mitochondrial dysfunction, **characterized in that** said composition is an orally administrable composition containing L-arginine as an active ingredient.

## Description

### (TECHNICAL FIELD)

The present invention relates to a composition for preventing and/or treating the expression of clinical symptoms in a disease caused by mitochondrial dysfunction, which composition comprises L-arginine as an active ingredient and is to be administered orally.

### (BACKGROUND ART)

Mitochondria, one kind of subcellular organelles, contain a mitochondrial DNA which carries its own genetic information, and the main function thereof is to produce energy. When abnormalities are developed owing to the mutation of this mitochondrial DNA in the electron transfer system of mitochondria, an energy production amount is decreased, which leads to dysfunction of all the cells and tissues throughout the body. This dysfunction is marked particularly in the neutral nervous system, skeletal muscles, cardiac muscles, and the like, which have a high energy demand.

Various diseases due to mitochondrial dysfunction are considered to occur most frequently among human hereditary diseases. Most of them are developed in childhood, and their types range widely from short statue and easy fatigability even to death. Mitochondrial dysfunction sometimes starts with aging.

Diseases caused by mitochondrial dysfunction include MELAS (mitochondrial myopathy, encephalopathy, lactic acidosis, and stroke-like episodes), CPEO (chronic progressive external ophthalmoplegia), and MERRF (myoclonus epilepsy associated with ragged-red fibers). Of these mitochondrial diseases, MELAS occurs most frequently and it makes up about 25% of all the mitochondrial diseases. With regards to the symptoms, for example, in MELAS, after repetition of cerebral apoplexy-like episodes, consciousness disorder, movement disorder, aphasia, hemianopsia or the like occurs. The recurrence makes such symptoms more serious. Intelligence disabilities come next and many patients are at last confined to bed. Some patients with severe symptoms die after the elapse of several years.

Diseases caused by mitochondrial dysfunction lead to insufficient energy in the cells and organs and accumulation of lactic acid owing to abnormalities in the energy production mechanism of mitochondria. Lactic acidosis due to the accumulation of lactic acid is treated by intravenous administration of an alkali agent such as sodium bicarbonate liquid, infusion for the purpose of accelerating excretion of the lactic acid, or the like. Alternatively, the accumulation of lactic acid is prevented by a low carbohydrate diet, while metabolism of lactic acid is promoted by the administration of vitamin B₁, biotin and sodium dichloroacetate ("Nippon Rinsho", 60, Supplement 4 "Mitochondria and Mitochondrial Diseases").

There is also a case where riluzole (2-amino-6-trifluoromethoxybenzothiazole), that is, a therapeutic drug for ALS (amyotrophic lateral sclerosis), is used against diseases caused by mitochondrial dysfunction (Japanese Domestic Publication No. 1997-507498, of the translated version of a PCT application).

Although the diseases caused by mitochondrial dysfunction are serious, gene therapy for the fundamental treatment of them is not realistic at present.

Various medicaments have been tried for the treatment of the diseases caused by mitochondrial dysfunction, but there is no unified view of their effectiveness.

Particularly for the treatment of the expression of clinical symptoms of the diseases caused by mitochondrial dysfunction starting in childhood, there is a demand for the development and creation of new types of remedies which can be administered continuously for preventing worsening or progression of the symptoms and have less side effects from the viewpoint of QOL (quality of life) of patients.

### (DISCLOSURE OF THE INVENTION)

The present inventors have made the pathological inspection of patients who developed in childhood MELAS with symptoms such as paroxysmal headache, vomiting, one-sided convulsion and the like, out of various types diseases caused by mitochondrial dysfunction, and observed, by a muscle biopsy, an abnormal accumulation image of mitochondria and abnormal staining of small and medium arterial walls. As a result, the present inventors have revealed that mitochondrial abnormalities existed in the skeletal muscles and the other organs. Moreover, from the fact that such abnormal staining of small and medium arterial walls was observed not only in the small and medium arteries in the muscles but also in the arteries in the central nervous system, they have also found that MELAS has vascular disorders.

Based on the fact that complete removal of active oxygen is not performed in abnormal mitochondria, the present inventors have set a hypothesis that cerebral apoplexy-like episodes of MELAS patients result from partial diastolic dysfunction of small arteries in the brain while taking into consideration, the possibility of mitochondria, which have been abnormally accumulated in the vascular smooth-muscle cells, producing free radicals attributable to tissue disorders and causing vascular disorders. The present inventors considered on the basis of this hypothesis that a medicament which is selected from various medicaments acting on the endothelial cells of small arteries in the brain and having an effect on the promotion of blood flow and is effective for improving disorders of the cerebral tissue caused by a blocked blood flow and lactic acidosis due to anaerobic glycolytic metabolism, is useful for the improvement of the clinical symptoms of MELAS patients.

Therapeutic agents having, as an active ingredient, an amino acid selected from the group consisting of arginine, leucine and isoleucine and glucose have so far been investigated in view of the fact that the brain damage can be treated by heightening the resistance against the glutamic acid in the brain when a drastic rise in the glutamic acid in the brain occurs by temporary or intermittent cerebral ischemia.

The present inventors have investigated the possibility of application of arginine to the treatment of the expression of clinical symptoms in the diseases caused by mitochondrial dysfunction, because various reports on the relationship between the physiological action of nitric oxide and diseases suggested that arginine, in particular, becomes a substrate for nitric oxide synthase in a blood vessel wall and has a vasodilator effect via nitric oxide.

As a result of intravenous drip infusion of L-arginine monohydrochloride to three patients including a 17-year old female who came to the hospital to treat her periodical vomiting, one-sided convulsion and short statue and was recognized to have 3243G mutation of mitochondria tRNA ^{(Leu)}, the present inventors have found that this method is effective for the treatment of the acute stage of cerebral apoplexy-like episode due to MELAS.

This treatment by intravenous injection is, however, not convenient for MELAS patients, because, in order to receive intravenous injection in the acute stage of cerebral apoplexy-like episode, they have to go to the hospital to receive such intravenous injection immediately after the episode has occurred. This treatment is, indeed, effective as a therapy when cerebral apoplexy-like episodes have appeared, but cannot improve the warning symptoms of the episode, particularly scintillating scotoma. The scintillating scotoma refers to an abnormal visual field which is one of warning symptoms of a cerebral apoplexy-like episode, and when it occurs, a riffle-like light suddenly appears in a part of the visual field and usually lasts for 5 to 30 minutes. Moreover, administration of a large amount of L-arginine monohydrochloride into a blood vessel may presumably cause side effects such as drop in blood pressure, hydrochloric acidosis, or the like.

On the other hand, expression of sudden cerebral apoplexy-like episodes worsens cerebral infarction and intelligence disabilities so that the most important problem for clinicians is how to prevent such episodes.

Particularly for the treatment of the expression of clinical symptoms in the disease caused by mitochondrial dysfunction which has started in childhood, there is a demand for the development and creation of a new-type therapeutic agent capable of treating-the expression of clinical symptoms in the disease caused by mitochondrial dysfunction while being administrable continuously for the purpose of stopping the worsening or progression of the symptoms and having less side effects from the viewpoint of QOL (Quality of Life) of patients.

The present inventors have therefore proceeded with a further investigation with a view to finding a safe and convenient treatment method capable of improving the acute-stage of an episode and warning symptoms thereof from which MELAS patients suffer.

As a result of having studied the administration route and dosage of L-arginine, the present inventors have surprisingly found that when L-arginine is administered orally at a low dosage maintenance therapy-wise, the frequency and degree of not only acute stage of episodes but also warning symptoms are remarkably reduced. Described specifically, the present inventors have found that, without employing the conventionally reported method in which L-arginine is administered rapidly in high dose (intravenous infusion of as much as 0.5 g/kg over 30 minutes) through intravenous injection, not only the warning symptoms of episodes, including scintillating scotoma, but also the frequency and degree of the acute stage of episodes can be reduced by oral administration of L-arginine at low dose. Based on these findings, the present invention has been completed.

Accordingly, the present invention relates to (1) a composition for preventing and/or treating the expression of clinical symptoms in a disease caused by mitochondrial dysfunction, characterized in that it is an orally administrable composition containing L-arginine as an active ingredient; (2) the composition of (1) as described above for preventing and/or treating the expression of clinical symptoms in the disease caused by mitochondrial dysfunction, characterized in that it is orally administered in an amount of from 1 to 30 g a day per adult in terms of L-arginine; (3) the composition of (1) or (2) as described above for preventing and/or treating the expression of clinical symptoms in the disease caused by mitochondrial dysfunction, characterized in that the disease caused by mitochondrial dysfunction is a cerebral apoplexy-like episode of MELAS or a warning symptom thereof; (4) the composition of (3) as described above for preventing and/or treating the expression of clinical symptoms in the disease caused by mitochondrial dysfunction, characterized in that the warning symptom of a cerebral apoplexy-like episode is scintillating scotoma; (5) the composition of any one of (1) to (4) as described above for preventing and/or treating the expression of clinical symptoms in the disease caused by mitochondrial dysfunction, characterized in that it contains, in addition to L-arginine, another mitochondrial function adjuvant such as a different amino acid, vitamin and the like and/or nitric oxide-releasing agent; and (6) the composition of any one of (1) to (5) as described above for preventing and/or treating the expression of clinical symptoms in the disease caused by mitochondrial dysfunction, characterized in that it contains, as an active ingredient, L-arginine in the free form and L-arginine monohydrochloride in combination.

The present invention will hereinafter be described more specifically.

L-arginine to be incorporated in the composition of the present invention is, of course, L-arginine not only in the free form but also in the form of a pharmaceutically acceptable salt. Accordingly, the term "L-arginine" as used herein embraces L-arginine in the free form and pharmaceutically acceptable salt thereof (L-arginine in the broad sense) unless otherwise understood from the context.

As such salts, there are mentioned acid addition salts and base salts. Examples of the pharmaceutically acceptable acid addition salts of L-arginine include the salt with an inorganic acid such as hydrogen chloride, hydrogen bromide, sulfuric acid, phosphoric acid or the like, and the salt with an organic acid such as acetic acid, lactic acid, citric acid, tartaric acid, maleic acid, fumaric acid, monomethylsulfuric acid or the like. Examples of the pharmaceutically acceptable base salts of L-arginine include the salt with an inorganic base such as sodium, potassium, calcium, ammonia, or the like, and the salt of an organic base such as ethylenediamine, propylenediamine, ethanolamine, monoalkylethanolamine, dialkylethanolamine, diethanolamine, triethanolamine or the like. The composition of the present invention embraces a dosage form composed only of L-arginine in the free form and/or in the form of its pharmaceutically acceptable salt (composition in the broad sense).

L-arginine to be incorporated as the active ingredient in a composition for oral administration of the present invention is preferably L-arginine in the free form, L-arginine in the form of the monohydrochloride thereof, or in the form of a mixture of both, in view of easy intake even by the aged or children, and prevention of side effects which will otherwise occur due to the long-term administration. Particularly upon oral administration, use of L-arginine monohydrochloride is preferred because it does not have a bad taste. Incorporation of L-arginine in the free form and L-arginine monohydrochloride at an equimolar amount is, however, preferred in order to prevent acidosis derived from the hydrochloride.

Regarding the amount to be administered, of a composition for oral administration of the present invention, it is administered in an amount of from 1 to 50 g, preferably from 1 to 30 g/day per adult, in terms of L-arginine in the free form. It is particularly recommendable to administer L-arginine in the free form and its monohydrochloride form at an equimolar amount or in the form of an equimolar mixture of both in an amount of from 1 to 30 g/day, in terms of L-arginine, per adult in at least two portions. When the amount is below the above described range, L-arginine does not bring about its effect fully. Administration of amounts exceeding the above-described range is, on the other hand, not preferred, because it will be a cause for deterioration in QOL or hydrochloric acidosis.

The composition for oral administration of the present invention may further contain an adjuvant for reinforcing the mitochondrial function. As such adjuvants there may be mentioned saccharides, intermediates for citric acid cycle (which may be also called "TCA cycle", "tricarboxylic acid cycle", or "Krebs cycle"), precursors for citric acid cycle intermediates or salts thereof, vitamins, amino acids, minerals, antioxidants, metabolism improvers and the like.

Examples of the above-described saccharides include monosaccharides, disaccharides and polysaccharides. Specific examples include glucose, fructose, mannose, galactose, sucrose, maltose, lactose, starch, and the like.

Examples of the citric acid cycle intermediates include citric acid, aconitic acid, isocitric acid, α-ketoglutaric acid, succinic acid, fumaric acid, malic acid, oxaloacetic acid, and the like.

Examples of the precursors for citric acid cycle intermediates or salts thereof include 2-keto-4-hydroxypropanol, 2,4-dihydroxybutanol, 2-keto-4-hydroxybutanol, 2,4-dihydroxybutyric acid, 2-keto-4-hydroxybutyric acid, aspartates, monoalkyl oxaloacetates, dialkyl oxaloacetates, mono- or di-alkyl citrates, aconitates, isocitrates, α-ketoglutarates, succinates, fumarates, malates, oxaloacetates, and the like.

Examples of the vitamins include Vitamin B₁, Vitamin B₂, Vitamin B₆, Vitamin B₁₂, Vitamin C, Vitamin A, Vitamin D, Vitamin E, Vitamin K, Vitamin H, folic acid, pantothenic acids, and nicotinic acids. Vitamin C and Vitamin E are especially preferred adjuvants rich in the properties as an antioxidant.

No particular limitation is imposed on such amino acids insofar as they are usually employed for infusion. Specific examples include L-isoleucine, L-leucine, L-valine, L-lysine, L-methionine, L-phenylalanine, L-threonine, L-tryptophan, L-alanine, L-arginine, L-aspartic acid, L-cysteine, L-glutamic acid, L-histidine, L-proline, L-serine, L-tyrosine, glycine, and the like. Although these amino acids may be used (incorporated) either singly or in combination, use of a plurality of them in combination is preferred. Especially, use of eight essential amino acids, that is, L-tryptophan, L-methionine, L-lysine, L-phenylalanine, L-leucine, L-isoleucine, L-valine and L-threonine in combination is preferred, and use of eight essential amino acids and a non-essential amino acid in combination is more preferred. Use of branched amino acids, that is, L-valine, L-isoleucine and L-leucine as an adjuvant is especially preferred from the standpoint of storage stability.

No particular limitation is imposed on the mineral insofar as it is usually employed in this field, for example, as an infusion component. Specific examples include calcium, sodium, potassium, magnesium, chlorine and phosphorus in the form of an inorganic or organic salt. As such inorganic or organic salts, the same salts as incorporated as an active ingredient in an infusion or enteral nutrient which has already been put on the market, can be used. A trace element can also be added as the mineral. The term "trace element" means a metallic element indispensable for an organism though in only trace amounts. Incorporation of a trace element as an adjuvant is especially preferred because it heightens the activity of an enzyme in the mitochondria. Specific examples include zinc, iron, manganese, copper, chromium, molybdenum, selenium, fluorine and iodine in the form of an inorganic or organic salt. In this case, it is needless to say that a trace element preparation for high calorie infusion already put on the market can be incorporated. Each trace element may be added in consideration of a daily requirement.

A chemical capable of continuously releasing nitric oxide under physiological conditions (which will hereinafter be abbreviated as "nitric oxide-releasing agent") may be added to a composition for oral administration of the present invention. For example, nitro group-containing compounds called "nitrate agent" or "nitro agent" are embraced in such chemicals capable of continuously releasing nitric oxide.

Examples of such nitrate agents include sodium nitroprusside, nitroglycerin, glyceryl trinitrate, isosorbide mononitrate, isosorbide dinitrate, molsidomine and S-nitroso-N-acetyl-DL-penicillamine. Further examples of the nitric oxide-providing agent include dithiothreitol, cysteine, N-acetylcysteine, mercaptosuccinic acid, thiosalicylic acid, methylthiosalicylic acid, and the like.

The composition for oral administration of the present invention is prepared in an appropriate dosage form such as powders, fine granules, granules, tablets, capsules and internal medicines or the like. Among these dosage forms, preferred are fine granules or granules modified to have good taste and smell, in order to be easily taken by the aged and children who tend to have troubles in swallowing.

These formulations can be each prepared in a conventional manner by using L-arginine (in the free form or/and in the form of a pharmaceutically acceptable salt thereof) as it is; by mixing it with pharmacologically or pharmaceutically acceptable additive(s) according to its dosage form, followed by pulverizing the mixture; by dissolving it in a proper solvent, followed by emulsifying or suspending the solution; mixing it with a proper base; or the like.

Examples of the additives to be added to powders, fine granules, granules, tablets, capsules or the like include excipients (such as lactose, glucose, D-mannitol, starch, crystalline cellulose, calcium carbonate, kaolin, light silicic anhydride, trehalose, and the like), binders (such as starch paste solution, gelatin solution, hydroxypropyl cellulose, hydroxypropylmethyl cellulose, polyvinylpyrrolidone, and the like), disintegrants (such as starch, gelatin powder, carboxymethyl cellulose, carboxymethylcellulose calcium salt, and the like), lubricants (such as magnesium stearate, talc, and the like), coating agents (such as hydroxypropyl cellulose, hydroxypropylmethyl cellulose, acetyl cellulose, sucrose, titanium oxide, and the like), and the like. If necessary, colorants, taste corrigents, smell corrigents and the like are added. Examples of the additives to be added to the internal medicine include preservatives (such as benzoic acid, paraoxybenzoic acid esters, sodium dehydroacetate, and the like), suspending or emulsifying agents (such as gum arabic, tragacanth gum, carboxymethylcellulose sodium salt, methyl cellulose, yolk lecithin, surfactant, and the like), sweeteners (such as trehalose, citric acid, and the like), and the like. Colorants, stabilizers and the like are also added further as needed. The solvent used for the internal medicine is mainly purified water, but ethanol, glycerin, propylene glycol or the like can also be used instead. As a suitable base, polyethylene glycol or the like can be mentioned for example.

L-arginine (either in the free form or in the form of a pharmaceutically acceptable salt thereof) is stimulating to the stomach walls, and therefore, it is preferably prepared as an entericcoated preparation. As enteric coating substances usable here, there may be mentioned those substances which are substantially insoluble in water of pH 4 or less but soluble in a solution having a pH of 4.5 or greater, especially a pH from 5.5 to 7.5. Substances ordinarily used for entericcoated preparations such as cellulose derivatives, dibasic acid esters of cellulose or polyvinyl compound, acrylic acid-based copolymers and maleic acid-based copolymers can be used for the inventive prupose.

Examples of the cellulose derivatives include' carboxymethylethyl cellulose, and the like.

Examples of the dibasic acid esters of cellulose or polyvinyl compound include cellulose·acetate·phthalate, cellulose·acetate·succinate, cellulose·acetate·maleate, hydroxypropylmethyl cellulose·phthalate, hydroxypropylmethyl cellulose·acetate·siccinate, polyvinylacetate·phthalate, polyvinyl propionate·phthalate, polyvinyl butylate·phthalate, and the like.

Examples of the acrylic acid-based copolymers include methyl acrylate·methacrylic acid copolymer, ethyl acrylate·methacrylic acid copolymer, methyl methacrylate-methacrylic acid copolymer, and the like.

Examples of the maleic acid-based copolymers include vinyl acetate·maleic anhydride copolymer, styrene-maleic acid copolymer, and the like.

As the above-described enteric coating substances, commercially available ones are also suited. For example, "HP-50" or "HP-55" (each, ex Shin-etsu Chemical) and the like can be used as the hydroxypropylmethyl cellulose·phthalate. As the methyl methacrylate·methacrylic acid copolymer, "EUDRAGIT L" or "EUDRAGIT S" (each, ex Röhm Pharma (Germany)) and the like can be used.

L-arginine (in the free form or in the form of a pharmaceutically acceptable salt thereof) serving as an active ingredient of the composition for oral administration of the present invention is colored and decomposed with the passage of time because it causes Maillard reaction, for example, with glucose or sucrose. Starch, trehalose or the like which does not cause Maillard reaction is therefore preferred as an additive. More preferably, use of trehalose as an additive for the preparation of the entericcoated preparation is preferred.

Such L-arginine in the free form or in the form of a pharmaceutically acceptable salt thereof is commercially available as a pharmaceutical, for example, "Arginine Injection Morishita", an injection solution of arginine hydrochloride as defined in the Japanese Pharmacopoeia to be used for pituitary function test, "Argi-U granules", a drug for urea cycle disorder, and "Argi-U injection", a drug to be used for the same purpose (each, ex Aninomoto Pharma). Their toxicity data can be found in many publications and there is no particular problem about their safety.

### (Best Mode for Carrying Out the Invention)

The present invention will hereinafter be described in further detail by examples.

### (1a) Clinical Test (1)

To three 12-31 year-old patients diagnosed as MELAS and recognized to indicate various paroxysmal abnormalities, 2.6 g (2 g in terms of L-arginine) of "Argi-U granules" (ex Ajinomoto Pharma), a granular preparation containing, as an active ingredient, an equimolar mixture of L-arginine and L-arginine monohydrochloride was administered three times a day (corresponding to 6 g of L-arginine as a daily dose) and expression frequencies of cerebral apoplexy-like episodes and warning symptoms thereof before and after administration were compared.

As a result, it has been found that in each of the three patients, the expression frequencies of the episodes and warning symptoms were decreased. In particular, the onset frequencies of serious one-sided convulsions requiring treatment in the hospital showed a marked decrease. In addition, by the administration of the above-described granules just after the awareness of scintillating scotoma, one of the warning symptoms, the symptom weakened in about 30 minutes after the administration and, together with a decline in the onset frequencies of this warning symptom, speedy improvement in this symptom was recognized.

### (1b) Clinical Test (2)

To two female patients who were 22 and 47 years old, respectively, diagnosed as MELAS and recognized to indicate paroxysmal abnormalities, 2.6 g (2 g in terms of L-arginine) of "Argi-U granule" (ex Ajinomoto Pharma), a granular preparation containing, as an active ingredient, an equimolar mixture of L-arginine and L-arginine monohydrochloride was administered three times a day (corresponding to 6 g of L-arginine as a daily dose) and expression frequencies of cerebral apoplexy-like episodes and warning symptoms thereof before and after administration were compared, as in the above-described Clinical Test (1).

As a result, it has been found that in each of these two patients, the onset frequencies of the episodes and warning symptoms were decreased (the frequencies of the episodes and warning symptoms per month were 1.7 times per month for one patient and 1.0 times per month for the other parient, respectively, before administration, which were remarkably decreased to 0.2 times per month and 0.3 times per month, respectively). In particular, the occurrence frequencies of one-sided convulsion serious enough to require treatment in the hospital showed a marked decrease.

### (2) Preparation Example of an Entericcoated Preparation

Using a centrifugal flow type granulate-coating equipment "CF-360" (ex Freund Industries), a water-ethanol solution having, suspended therein, 200 g of a 7:3 powder mixture of hydroxypropylmethyl cellulose acetate succinate and talc as an enteric coating substance was sprayed onto 200 g of spherical core particles having an average particle size of from 1,000 to 500 µm, which particles had been obtained in advance by mixing and granulating an equimolar mixture of free L-arginine and L-arginine monohydrochloride, starch and hydroxypropyl cellulose, while rolling the particles and sending warm air thereto. After further rolling and drying, the coated granules were cooled to room temperature, whereby 350 g of an entericcoated preparation was obtained.

An elution test of the resulting entericcoated preparation was conducted using 900 ml of each of the Japanese Pharmacopoeia Solution No. 1 (pH 1.2) and Solution No. 2 (pH 6.8) as the solvent under the condition of 37°C and a paddle rotation speed of 100 rpm. For the Solution No.1, the elution of the entericcoated preparation thus obtained was suppressed, while for the Solution No. 2, the elution was smooth. This suggests that the preparation has an enteric coating function.

### (3) Preparation Example of a Liquid Preparation

L-arginine, Vitamin C, Vitamin E, citric acid, succinic acid, orange flavor, saccharin sodium and sodium hydrogen sulfite were weighed in accordance with the amounts as shown below in Table 1, and dissolved in purified water. The resulting solution was filled in a bottle made of a multilayered resin not permitting smooth permeation of oxygen and having a substantial capacity of 350 ml. After the bottle was hermetically sealed with a mouth member, it was sterilized to prepare a bottled liquid preparation. The resulting bottled liquid preparation can be administered rightly after the expression of warning symptoms of an episode and has an immediate effect. The liquid preparation is therefore a preparation for oral administration excellent in convenience and suitable for domiciliary maintenance therapy of MELAS patients.

**Table 1:**

| Amounts to be added per 350 ml | |
|---|---|
| L-arginine Vitamin C | 10.000 g 0.500 g |
| Vitamin E | 0.440 g |
| Citric acid | 0.440 g |
| Succinic acid | 0.440 g |
| Saccharin sodium | 0.200 g |
| Sodium hydrogen nitrite | 0.070 g |
| Orange flavor | q.s. |

### (Industrial Applicability)

In a composition of the present invention for preventing and/or treating the expression of clinical symptoms in a disease caused by mitochondrial dysfunction, which composition is to be orally administered, the L-arginine serving as the active ingredient thereof can be a substrate for a nitrogen monoxide synthase in the blood vessel walls, and is, owing to a vasodilator action via nitric oxide, effective for the treatment of the expression of clinical symptoms in a disease caused by mitochondrial dysfunction. For example, it can improve the episode symptoms of MELAS patients and also warning symptoms thereof in general. The composition for oral administration of the present invention can be administered safely and conveniently. Particularly, maintenance therapy at home is necessary for preventing the recurrence of episode symptoms of patients at the initial onset stage who visit the hospital to treat the cerebral apoplexy-like episode. The preventing and/or treating composition of the present invention is therefore remarkably beneficial for maintenance therapy.

## Claims

1. A composition for preventing and/or treating the expression of clinical symptoms in a disease caused by mitochondrial dysfunction, **characterized in that** said composition is an orally administrable composition containing L-arginine as an active ingredient.

2. The composition of Claim 1 for preventing and/or treating the expression of clinical symptoms in a disease caused by mitochondrial dysfunction, **characterized in that** said compostion is orally administered in an amount of from 1 to 30 g a day per adult in terms of L-arginine.

3. The composition of Claim 1 or 2 for preventing and/or treating the expression of clinical symptoms in a disease caused by mitochondrial dysfunction, **characterized in that** said disease caused by mitochondrial dysfunction is a cerebral apoplexy-like episode of MELAS or a warning symptom thereof.

4. The composition of Claim 3 for preventing and/or treating the expression of clinical symptoms in a disease caused by mitochondrial dysfunction, **characterized in that** said warning symptom of a cerebral apoplexy-like episode is' scintillating scotoma.

5. The composition of any one of Claim 1-4 for preventing and/or treating the expression of clinical symptoms in a disease caused by mitochondrial dysfunction, **characterized in that** said compostion contains, in addition to L-arginine, another mitochondrial function adjuvant such as a different amino acid, vitamin and the like and/or nitric oxide-releasing agent.

6. The composition of any one of Claim 1-5 for preventing and/or treating the expression of clinical symptoms in a disease caused by mitochondrial dysfunction, **characterized in that** said compostion contains, as an active ingredient, L-arginine in the free form and L-arginine in its monohydrochloride form in combination.
